Europäisches Patentamt

European Patent Office

Office européen des brevets

(11) Publication number: **0 092 163**
**B1**

(12) **EUROPEAN PATENT SPECIFICATION**

(45) Date of publication of patent specification: **28.09.88**

(21) Application number: **83103582.9**

(22) Date of filing: **14.04.83**

(51) Int. Cl.⁴: **C 12 P 21/00,** C 07 K 15/00,
A 61 K 37/02

(54) Purification of interleukin 2.

(30) Priority: **20.04.82 US 370223**

(43) Date of publication of application:
**26.10.83 Bulletin 83/43**

(45) Publication of the grant of the patent:
**28.09.88 Bulletin 88/39**

(84) Designated Contracting States:
**AT BE CH DE FR GB IT LI LU NL SE**

(56) References cited:
**EP-A-0 089 062**
**DE-A-3 149 360**

**CHEMICAL ABSTRACTS, vol. 100, no. 1, 2nd
January 1984, page 389, abstract 4465m
Columbus, Ohio, US U. SCHWULERA et al.:
"Removal of phytohemagglutin from human
interleukin-2 containing supernatants by dye
matrex ligand chromatography in comparison
to other methods"**

(73) Proprietor: **Sloan-Kettering Institute For Cancer
Research
1275 York Avenue
New York New York 10021 (US)**

(72) Inventor: **Mertelsmann, Roland
301 Millwood Road
Chappaqua New York 10514 (US)**
Inventor: **Welte, Karl
504 East 81 Street
New York New York 10028 (US)**
Inventor: **Venuta, Salvatore
Via Cilea 183
I-80127 Napoli (IT)**

(74) Representative: **Patentanwälte Schulze Horn
und Hoffmeister
Goldstrasse 36
D-4400 Münster (DE)**

(56) References cited:

**CHEMICAL ABSTRACTS, vol. 97, no. 17, 25th
October 1982, page 516, abstract 142913b
Columbus, Ohio, US K. WELTE et al.:
"Purification of human interleukin 2 to a
apparent homogeneity and its molecular
heterogeneity"**

Courier Press, Leamington Spa, England.

(56) References cited:
THE JOURNAL OF IMMUNOLOGY, vol. 128 no.
3, March 1982, pages 1122-1127 J.W. MIER et
al.: "The purification and properties of human T
cell growth factor"

THE JOURNAL OF IMMUNOLOGY, vol. 128, no.
4, April 1982, pages 1620-1624 B.M. STADLER
et al.: "Monoclonal antibody against human
interleukin 2 (IL 2). I. Purification of IL 2 for the
Production of Monoclonal Antibodies"

THE JOURNAL OF IMMUNOLOGY, vol. 127, no.
6, December 1981, pages 2361-2365, US M.B.
FRANK et al.: "Biochemical and biologic
characterization of lymphocyte regulatory
molecules. VIII. Purification of interleukin 2
from a human T cell Leukemia"

THE JOURNAL OF IMMUNOLOGY, vol. 124 no.
4, April 1980, pages 1954-1962, US S. GILLIS et
al.: "Biochemical characterization of
lymphocyte regulatory molecules. II.
Purification of a class of rat and human
lymphokines"

THE JOURNAL OF IMMUNOLOGY, vol. 126, no.
4, April 1981, pages 1351-1354, US B. CAPLAN
et al.: "Properties of sodium dodecyl sulfate-
denatured interleukin 2"

CHEMICAL ABSTRACTS, vol. 100, no. 1, 2nd
January 1984, page 389, abstract 4465m
Columbus, Ohio, US U. SCHWULERA et al.:
"Removal of phytohemagglutin from human
interleukin-2 containing supernatants by dye
matrex ligand chromatography in comparison
to other methods"

CHEMICAL ABSTRACTS, vol. 97, no. 17, 25th
October 1982, page 516, abstract 142913b
Columbus, Ohio, US K. WELTE et al.:
"Purification of human interleukin 2 to a
apparent homogeneity and its molecular
heterogeneity"

## Description

This invention was made with support in part under Grant PO1—CA—20194, awarded by the National Cancer Institute, National Institute of Health, DHEW. The government has certain rights in this invention.

Interleukin 2 (IL 2; T-cell growth factor), produced with and without costimulation by Burkitt's lymphoma line Daudi, is purified approximately 37,000-fold to apparent homogeneity from lymphocyte-conditioned medium by $(NH_4)_2SO_4$-precipitation, ion-exchange chromatography (diethylaminoethyl cellulose), gel filtration (AcA 44 Ultrogel®), and hydrophobic chromatography, preferably on Blue Agarose and on Procion®-Red Agarose. Native IL 2 produced in the absence of Daudi cells has a molecular weight of about 26,000 daltons as measured by gel filtration and yields IL 2 having two molecular weights of about 16,000 and 17,000 daltons after denaturation as measured by sodium dodecyl sulfate-polyacrylamide gel electrophoresis. IL 2 produced in the presence of Daudi cells ($10^6$/ml) shows a molecular weight of approximately 14,500 daltons as measured by both gel filtration and sodium dodecyl sulfate-polyacrylamide gel electrophoresis.

The purified IL 2 shows a specific activity of about $10^6$ U/mg which appears to be independent of molecular weight and an isoelectric point of approximately pH 6.7 for the 26,000 MW form and about pH 8.1 for the 14,500 MW variant.

The purified IL 2 lacks detectable Interferon (alpha and gamma), granulocyte-macrophage colony-stimulating factor, T-cell replacing factor, and thymocyte differentiating activity and was free of any contaminating proteins as judged by silver staining and by $I^{125}$ exolabelling in sodium dodecyl sulfate-polyacrylamide gel electrophoresis. All four molecular forms of IL 2 were biologically active at concentrations of $10^{-11}$—$10^{-10}$ M ($0.2\pm0.05$ U/ml), supporting the growth of human and murine cytotoxic T-cell lines.

In recent years, several functionally and biochemically unique soluble proteins have been discovered that play a central role in regulating the responsiveness of the immune system and/or act as antigen-non-specific effector molecules capable of mediating one or more aspects of immune function. Most of these factors are synthesized by hemopoietic cells, especially lymphoid cells and monocytes, and are generally termed cytokines. Recent refinements in protein chemistry and separation techniques, and the development of continuous cell lines secreting particular cytokines, have made detailed cytokine analysis possible.

It was discovered that many of the cell interactions involved in the afferent arm of immune responses involved soluble helper or suppressor substances elaborated by these cells. These non-antibody mediators produced by lymphocytes and other cells generated a degree of interest comparable to that previously only given to antibodies themselves.

Today, there is hardly any area of the immune response concerning which cytokine action has not been demonstrated or inferred.

Soon after the discovery that plant lectins stimulated the proliferation of human T-lymphocytes, soluble mitogenic factors were found in the culture supernatants. Since 1965, many such mitogenic factors have been decribed, and, depending upon the assay used, as many different names and acronyms were appled to the activities.

In 1976, it was reported that conditioned media from lectin-stimulated mononuclear cells contained a mitogenic factor that would support the continuous exponential growth of lectin-activated human T-cells. This discovery allowed for the construction of a rapid, quantitative assay for the T-cell growth factor (TCGF, IL 2), in that the growth of the cultured T-cells was entirely dependent upon an exogenous supply of IL 2. Within 24 hours, sigmoid IL 2 dependent dose-response curves, which were amenable to probit analysis, yielded reproducible, quantitative data, such that a comparison of IL 2 titers was possible. The development of an IL 2 assay, which was rapid, simple and quantitative, prompted experiments to define its biochemical characteristics. As a result, it is now clear that IL 2 provides the mitogenic stimulus after lectin or antigen initiated T-cell activation. The addition of a lectin or antigen to mononuclear cells results in at least three responses: first, release of a soluble factor from monocytes/macrophages (IL 1); second, under the influence of this monokine, the release of IL 2 by a specific T-cell subset; and third, binding of IL 2 by separate T-cell subsets resulting in a proliferative response. While these responses all require initiation by lectins or antigens, the proliferative response is mediated solely by IL 2.

Interleukin 2 (IL 2, T-cell growth factor), discovered by Morgan et al. (Morgan, D. A., F. W. Ruscetti and R. C. Gallo, Selective In Vitro Growth of T-Lymphcytes from Normal Human Bone Marrows, Science 193:1007 (1976)) is produced by T-lymphocytes after antigen- or mitogen-stimulation and is required for the proliferation of activated T-cells. IL 2 is an essential mediator of the immune response (Paetkau, V., Lymphokines on the Move, Nature 294:689 (1981); Ruscetti, F. W., and R. C. Gallo, Human T-Lymphocyte Growth Factor: Regulation of Growth and Function of T-Lymphocytes, Blood 47:379 (1981)) and there is preliminary evidence that it may also be responsible for the abnormal cell proliferation in human lymphoblastic leukemias (Gillis, S., R. Mertelsmann, B. Clarkson and M. A. S. Moore, Correlation of Elevated Terminal Transferase Activity (TdT) with Production of T-Cell Growth Factor (TCGF) in Human Leukemia Cells, AACR, Abstract No. 955, p. 238 (1980); Venuta, S., R. Mertelsmann, K. Welte, S. P. Feldman, C. Y. Wang and M. A. S. Moore, Production and Regulation of Interleukin-2 in Human Lymphoblastic Leukemias Studied with T-Cell Monoclonal Antibodies (submitted)).

These observations have led investigators to initiate antigen-specific proliferative responses, followed by attempts to maintain functional, antigen-specific T-cells in continuous Il 2-dependent proliferation culture. The ability to culture functional monoclonal T-cells should provide the cellular reagents necessary for detailed studies of the nature of the T-cell immune response. The cultured T-cells have also provided the cellular reagents necessary for detailed studies of IL 2 cellular interaction.

Results of experimentation indicate that IL 2 satisfies most of the criteria for a hormone. It is produced by a distinct cell type and acts at a distance on other cell types by means of specific receptors. Study of the molecular mechanisms of IL 2 production, hormone-receptor interation, and the identification of IL 2 agonists and the antagonists should provide new insights into pathological diesease states which involve T-lymphocytes.

Studies of the mechanism of action of IL 2 using unpurified or partially purified preparations have been very difficult because conditioned media contain other lymphokines and cytokines with potent biological activities. Several groups have reported purification procedures for both murine (Watson, J., S. Gillis, M. Marbrook, D. Mochizuki and K. A. Smith, Biochemical and Biological Characterization of Lymphocyte Regulatory Molecules. I. Purification of a Class of Murine Lymphokines, J. Exp. Med. 150:849 (1979); Granelli-Piperno, A., J. D. Vassalli and E. Reich, Purification of Murine T-Cell Growth Factor. A Lymphocyte Mitogen with Helper Activity, J. Exp. Med. 154:422 (1981)) and human IL 2 (Mier, J. W., and R. C. Gallo, Purification and Some Characteristics of Human T-Cell Growthy Factor From Phytohemagglutinin-Stimulated Lymphocyte Conditioned Media, Proc. Natl. Acad. Sci. U.S.A. 77:6134 (1980); Gillis, S., K. A. Smith and J. Watson, Biochemical Characterization of Lymphocyte Regulatory Molecules. II. Purification of a Class of Rat and Human Lymphokines; J. Immunol. 124:1954 (1980); Robb, R. J., and K. A. Smith, Heterogeneity of Human T-Cell Growth Factor(s) Due To Variable Glycosylation, Mol. Immunol. 18:1087 (1981)). These IL 2 preparations have permitted an increasingly better definition of IL 2 regulation.

In the course of purification of human IL 2, several reproducible conditions for the production of high levels of human IL 2 have been previously developed. First, nylon-column purified peripheral blood lymphocytes prepared from nany allogeneic donors were cultured for 72 hours at $4 \times 10^6$ cells/ml in the presence of 1% phytohemagglutinin-M and a 0.25% bovine serum albumin. In general phytohemagglutinin-stimulated leukocytes from a single donor produce much lower levels of IL 2. Second is a method that relies on the use of phytohemagglutinin-stimulated cells from a single donor but co-cultivated with x-irradiated cells from a B-lymphoblastoid cell line such as RM—1, SR or Daudi. The rationale for use of B-cell lines was to provide a standard source of allogeneic stimulation and thus eliminate the need for mixing several allogeneic donors. Third, after screening for cell lines that will release IL 2 after mitogen stimulation, a human T-leukemic cell line Jurkat was found to be a high producer. This cell line has since been used by many investigators as a consistent source of IL 2. Tumour promoters have also been used as costimulators, leading to high IL 2 concentrations in the conditioned media, but these preparations would certainly be difficult to use in humans because of the potentially increased risk of cancer promotion.

In the past, IL 2 has been induced as crude lymphocyte-conditioned medium by any of the three methods previously described and prepared under serum-free conditions with bovine serum albumin (0.2%) as the serum replacement.

After ultrafiltration, the crude lymphocyte-conditioned medium was concentrated by $(NH_4)_2SO_4$ fractionation and then dialyzed. The dialyzed active sample was passed over an anion-exchange chromatographic column (diethylaminoethyl-Sepharose®). IL 2 activity eluted as a broad peak centered at approximately 0.07 M NaCl. Subsequent gel filtration with an Ultrogel AcA 54® column separated the IL 2 from most of the detectable proteins, and this sequence of steps achieved more than a 400-fold increase in specific activity over IL 2 in the serum-free lymphocyte-conditioned medium. The IL 2-containing material was further purified using polyacrylamide gel electrophoresis containing sodium dodecyl sulfate. The IL 2 activity corresponded to a pair of protein bands present in the 13,000 molecular weight region in the sodium dodecyl sulfate gel. This procedure has been reported by Mier and Gallo, J. Immunol. 128:1122 (1982), and Frank et al., J. Immunol. 127:2361 (1981), for the purification of human IL 2. More recently the use of phenylsepharose has been introduced as a first step in the purification scheme of IL 2 (Stadler et al., J. Immunol. 128:1620 (1982)). However, since phenylsepharose as hydrophobic absorption chromatography was used only in the first step of purification, the major advantages of this technique for separation of multiple lymphokines were not utilized (see below).

Thus although reports are known of purification procedures for both murine and human IL 2, and these preparations have permitted an increasingly better definition of IL 2 regulation, the purity of such preparations has not been well-documented.

We recently found that hydrophobic adsorption chromatography following other separation means permits the purification of human IL 2 to apparent homogeneity. A 37,000-fold purification may be achieved compared to the 60-fold purification reported by Stadler et al., J. Immunol. 128:1620 (1982), and the 800-fold purification disclosed by Mier et al., J. Immunol. 128:122 (1982). Our purified IL 2 preparation is apparently free of all other lymphokines and factors present in the crude lymphocyte-conditioned medium, including Interferon (alpha and gamma), colony stimulating factor (CSF), T-cell replacing factor, B-cell growth factor (BCGF), and serum thymic factor (FTS). The purified IL 2 obtained by us appears to be free of any contaminating proteins in sodium dodecyl sulfate-polyacrylamide gel electrophoresis after staining with a silver nitrate method (Merril, C. R., R. C. Switzer and M. L. Van Keuren, Trace Polypeptides in Cellular Extracts and Human Body Fluids Detected by Two-Dimensional Electrophoresis and a Highly Sensitive Silver Stain, Proc. Natl. Acad. Sci. U.S.A. 76:4335 (1979)), and after exolabelling with $I^{125}$ (Branca, A. A. and Baglioni, C., Evidence that Types I and II Interferon Have Different Receptors, Nature 294/768 (1981); Bio-Rad (Rockville Center, New York), Technical Bulletin 1071: Radio Iodinating Proteins with Enzymobeads (May 1981)). Up to three functionally active bands were detected in this preparation. Elution of the materials from the sliced gels possed high specific activity. We found that the molecular species of IL 2 are dependent on the experimental conditions used for IL 2 induction. All purification steps have been developed to allow large scale production and are currently being used for 10 liters of conditioned medium per week.

Although this purification protocol has been successfully used not only for IL 2 from normal lymphocytes, but also for IL 2 from leukemic lymphoblasts as well as from the Jurkat cell line, our work has focused predominantly on IL 2 from normal human lymphocytes. Since the pure material was intended for use in humans, leukemic cell sources as well as induction conditions using tumor promotors (Frank et al., J. Immunol. 127:2361 (1981); Stadler et al., J. Immunol. 128:1620 (1982) and/or tumour cell line costimulators (Gillis et al., J. Immunol. 124:1954 (1980)) appeared highly undesirable for this purpose. Furthermore, there is the possibility that the leukemic IL 2 might be different from normal IL 2 which, although unlikely, could be an additional hazard for in vivo administration.

Obtaining purified IL 2 according to the invention comprises conditioning the source medium to increase the IL 2 content thereof: concentrating the proteinaceous components of the medium by precipitating same and separating therefrom unprecipitated non-proteinaceous material and smaller molecules (e.g., MW<5,000), such as amino acids and small peptides; removing extra salts from the proteinaceous material via dialysis; separating some of the non-specific proteins from desired proteinaceous material by anion exchange; effecting separation by molecular weight of the IL 2-containing proteinaceous material by gel filtration; and separating IL 2, which is highly hydrophobic, from other lymphokines of about the same molecular weight via hydrohphobic chromatography.

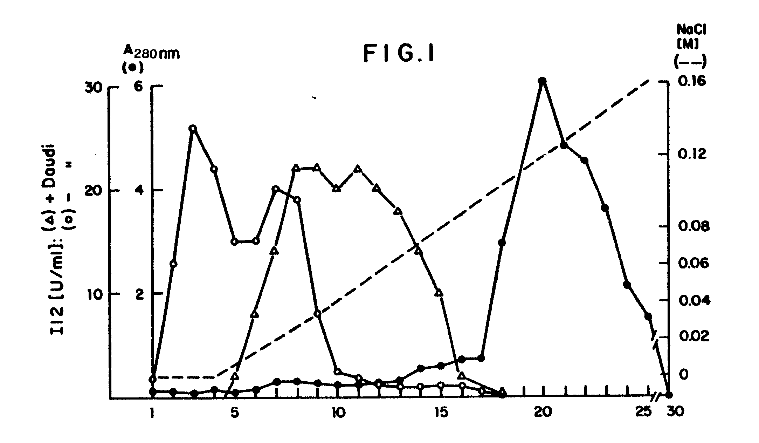

Fig. 1 relates to diethylaminoethyl cellulose (DE 52) chromatography of IL 2. A dialyzed sample of the $(NH_4)_2SO_4$-precipitate fraction was loaded on the DE 52 column. Proteins were eluted with a linear gradient of NaCL (0—0.3 M) in 0.05 M Tris-HCl (pH 7.8) and 5 ml fractions collected (absorbtion at 280 nm (●—●) IL 2 (U/ml produced in the presence (△—△) or absence (○—○) of Daudi cells).

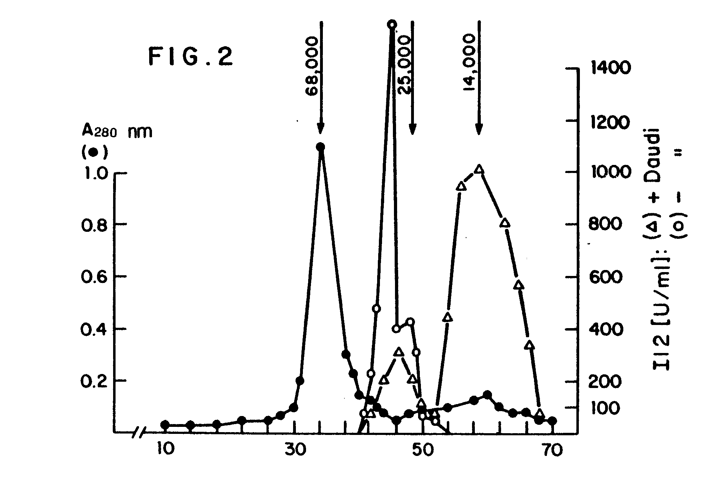

Fig. 2 concerns gel filtration of IL 2 on AcA 44 Ultrogel®. DE 52-purified IL 2 was loaded on an AcA 44 Ultrogel® column and eluted with phosphate-buffered saline (pH 7.2)/0.1% polyethyleneglycol (MW 6000). Six ml fractions were collected. The column was calibrated with bovine serum albumin (MW 68,000), chymotrypsinogen (MW 25,000), and ribonuclease A (MW 14,000) (absorption at 280 nm (●—●), IL 2 (U/ml produced in the presence (△—△) or absence (○—○) of Daudi cells).

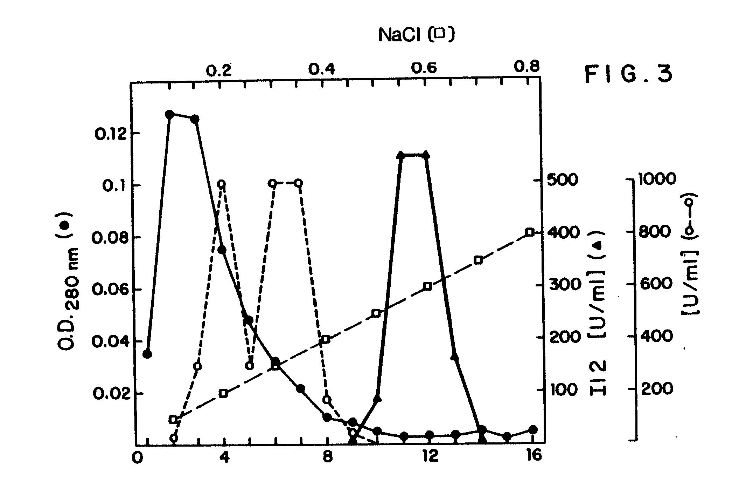

Fig. 3 illustrates chromatography of IL 2 on Blue Agarose. AcA 44 Ultrogel®-purified IL 2 was applied to a Blue Agarose column and eluted with a linear gradient of phosphate-buffered NaCl (0.05—0.8 M). Twenty ml fractions were collected (O.D. at 280 nm (●—●), IL 2 (U/ml) (▲—▲), alpha-Interferon (U/ml) (○—○), NaCl molarity (M) (□—□)).

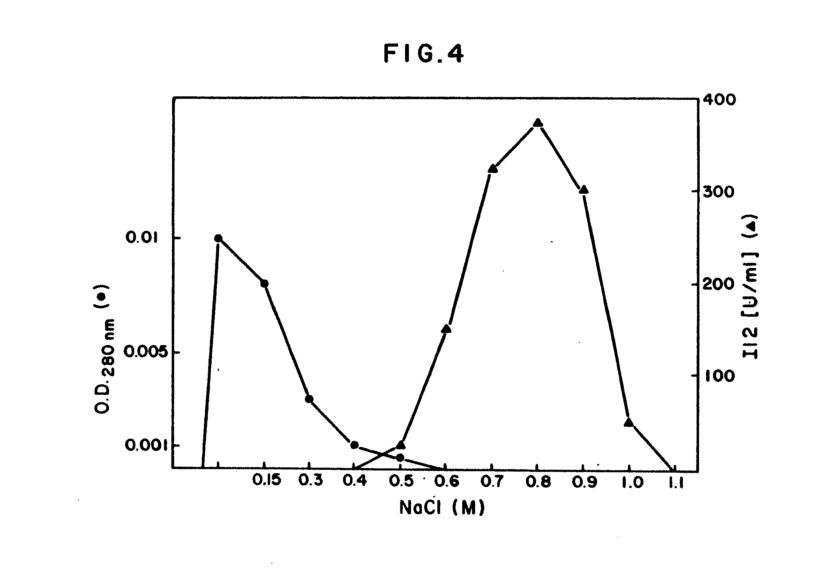

Fig. 4 relates to chromatography of IL 2 on Procion®-Red agarose. The IL 2-containing fractions from Blue Agarose were pooled and loaded on a Procion®-Red Agarose column. The bound proteins were eluted with a stepwise increase in salt concentration (0.15—1.0 M NaCl in phosphate buffer) (O.D. at 280 nm (●—●), IL 2 (U/ml) (▲—▲).

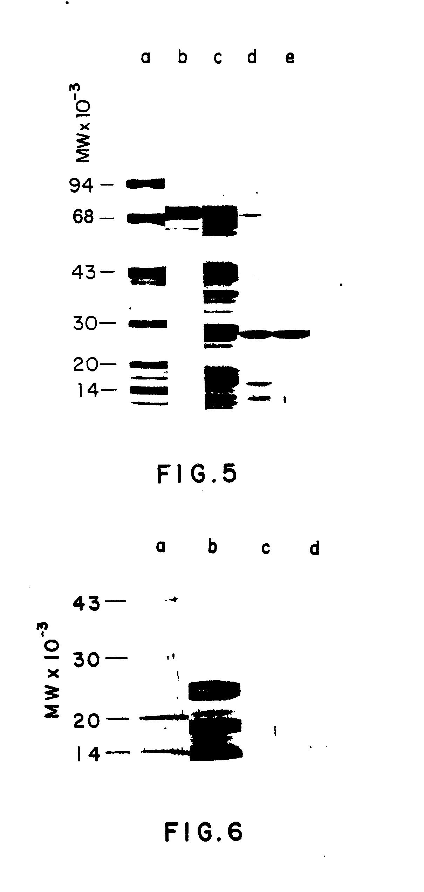

Fig. 5 illustrates a sodium dodecyl sulfate-polyacrylamide gel electrophoresis profile of various steps of IL 2 purification ((a) molecular weight standards: Phosphorylase b (MW 94,000), bovine serum albumin (MW 68,000), ovalbumin (MW 43,000), carbonic anhydrase (MW 30,000), soybean trypsin inhibitor (MW 20,000), and alpha-lactalbumin (MW 14,500); (b) IL 2-containing lymphocyte-conditioned medium; (c) dialyzed ammonium sulfate precipitate; (d) pool of IL 2-containing diethylaminoethyl cellulose eluate; (e) IL 2-containing fractions pooled from AcA 44 Ultrogel® gel filtration).

Fig. 6 shows the sodium dodecyl sulfate-polyacrylamide gel electrophoresis of Blue Agarose- and Procion®-Red Agarose-purified IL 2. IL 2 was treated with 2% sodium dodecyl sulfate and 5 mM 2-mercaptoethanol and applied to a 5—20% gradient gel. The protein bands were visualized by a silver nitrate method. The following marker proteins (200 ng each) were used: ovalbumin (MW 43,000), carbonic anhydrase (MW 30,000), soybean trypsin inhibitor (MW 20,000) and alpha-lactalbumin (MW 14,500 ((A) protein standards; (b) Blue Agarose-purified IL 2 produced in the absence of Daudi cells; (d Procion®-Red Agarose-purified IL 2 prepared in the absence of Daudi cells; (d) Procion®-Red Agarose-purified IL 2 obtained by costimulation with Daudi cells).

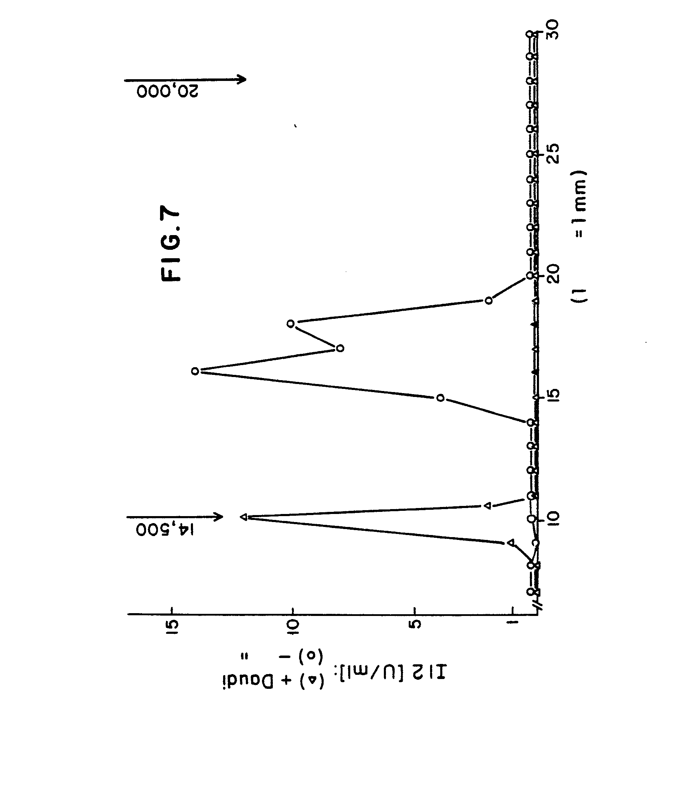

Fig. 7 shows the sodium dodecyl sulfate-polyacrylamide gel electrophoresis of Procion®-Red Agarose-purified IL 2 produced in the presence or absence of Daudi cells. The IL 2 preparations were treated with 2% sodium dodecyl sulfate and 5 mM 2-mercaptoethanol and applied to a 15% polyacrylamide gel. After electrophoresis, the gel was sliced into 1 mm sections and proteins eluted with 0.3 ml phosphate-buffered saline (pH 7.2). The eluted material was assayed for IL 2 activity. The arrows indicate the position of the protein standards soybean trypsin inhibitor (MW 20,000) and alpha-lactalbumin (MW 14,500) (IL 2 (U/ml) produced in the presence (△—△) or absence (○—○) of Daudi cells.

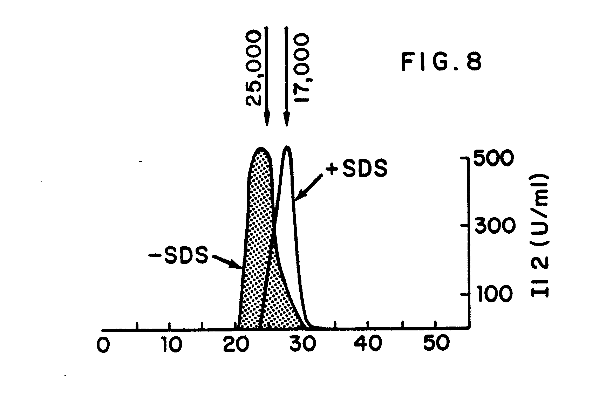

Fig. 8 relates to the gel purification chromatography of Blue Agarose-purified IL 2 on high performance liquid chromatography in the presence and absence of sodium dodecyl sulfate and dithiothreitol. IL 2, native or treated with 1% sodium dodecyl sulfate and 10 mM dithiothreitol, was applied to a high performance liquid chromatography gel filtration column. The following protein standards were used: bovine serum albumin (MW 68,000), ovalbumin (MW 43,000), chymotrypsinogen (MW 25,000), and ribonuclease A (MW 14,000). The arrows indicate the position of chymotrypsinogen and the calculated 17,000 daltons molecular weight region. The dotted area identifies the position of native IL 2 and the second peak corresponds to the sodium dodecyl sulfate-denatured IL 2.

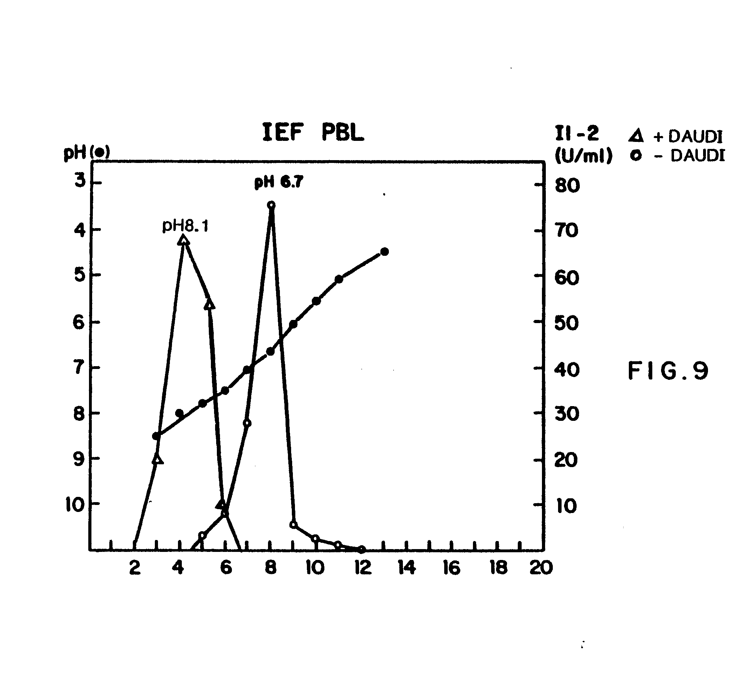

Fig. 9 concerns the isoelectrofocusing of AcA 44 Ultrogel®-purified IL 2. An isoelectrofocusing column (110 ml) was prepared by loading a 5—60% glycerol density gradient containing 2% Ampholines of pH 3.5—10. IL 2 eluted from AcA Ultrogel® was supplemented with 2% Ampholines (pH 3.5—10) and 20% glycerol and was layered onto the isodense region of the column gradient. Constant power was then applied to the column with a terminal voltage of 2000 V and a current of 5 mA (24 hr, 4°C) (IL 2 (U/ml) (O—O), pH gradient (●—●)).

### Preparation of Lymphocyte-Conditioned Medium (Fraction I)

Human lymphocytes were obtained from peripheral blood of multiple donors at New York Blood Center. In a typical procedure, the cells were initially stimulated by Sendai Virus ($10^4$ U/ml) as part of a protocol to induce Interferon. Twelve hours later the culture medium, rich in alpha-Interferon, was removed by centrifugation (800 × g). The cells were resuspended to $4 \times 10^6$/ml in serum-free RPMI 1640 (obtained from Gibco, Grand Island) supplemented with 0.25% bovine serum albumin (obtained from Sigma, St. Louis, Missouri) and 1% phytohemagglutinin-M (obtained from Gibco) and incubated at 37°C for 48 hours. In some preparations irradiated (5000 rad) Daudi cells ($10^6$/ml) were added to the medium in order to increase IL 2 production. At the end of the incubation, cells and cell debris were separated from the conditioned medium by centrifugation (10,000 × g, 15 min) and the supernatant was used for purification of IL 2).

### Ammonium Sulfate Precipitation (Fraction II)

1683 gm $(NH_4)_2SO_4$ were added to 3 liters of lymphocyte-conditioned medium to achieve 80% saturation. After gentle stirring overnight at 4°C, the precipitate was spun down (10,000 × g, 15 min), dissolved in 0.05 M Tris-HCl (pH 7.8) in a final volume of 300 ml, and subsequently dialyzed against 50 volumes of 0.05 M Tris-HCl buffer (pH 7.8) for 48 hours with five changes of the dialyzing buffer.

### Anion Exchange Chromatography (Fraction III)

For analytical purposes, 10 ml of the dialyzed concentrate was loaded on a 40 ml column of microgranular diethylaminoethyl cellulose (DE 52, obtained from Whatman, England) which had been previously equilibrated with 0.05 M Tris-HCl (pH 7.8). The column was washed with 80 ml of the same buffer and proteins, including IL 2, were eluted using a linear gradient of Tris-buffered NaCl (0—0.3 M NaCl) and 5 ml fractions were collected. IL 2-containing fractions were pooled and dialyzed against phosphate-buffered saline (pH 7.2) containing polyethyleneglycol (MW 6000) (50%, w/v) in order to concentrate pooled fractions. For large scale purification, 300 ml of diethylaminoethyl-cellulose slurry, equilibrated with 0.05 M Tris-HCl (pH 7.8) was gently mixed with 300 ml of redissolved and dialyzed ammonium sulfate precipitate. After 30 minutes the diethylaminoethyl cellulose was spun down and the supernatant saved (*Supernatant 1*). The pellet was resuspended in 300 ml of 0.05 M Tris-HCl (pH 7.8) containing 0.01 M NaCL. After 10 minutes the diethylaminoethyl cellulose was spun down again. (1,000 × g) and the resulting supernatant pooled with *Supernatant 1*. The pooled supernatants were concentrated by dialysis against polyethyleneglycol (MW 6000)/phosphate-buffered saline (pH 7.2) as described above.

### Gel Filtration (Fraction IV)

The concentrated diethylaminoethyl cellulose preparation was applied in 10 ml aliquots to an AcA 44 Ultrogel (obtained from LKB, Rockland, Maryland) column (2.5 × 90 cm), which had been previously equilibrated with phosphate-buffered saline (pH 7.2) containing 0.1% polyethyleneglycol (MW 6000). The flow rate was adjusted to 30 ml/hr and 6 ml fractions were collected. IL 2-containing fractions were pooled. The column was calibrated with bovine serum albumin (MW 68,000), chymotrypsinogen (MW 25,000), and ribonuclease A (MW 14,000), all obtained from Pharmacia (Piscataway, New Jersey). Subsequent to the preceding work, we have come to prefer the AcA 54 column over the AcA 44.

### Chromatography on Blue Agarose (Fraction V)

Two hundred ml of the active fractions pooled from the AcA 44 Ultrogel® column were applied to a Blue Agarose column (obtained from BRL, Gaithersburg, Maryland) mwith a bed volume of 40 ml that had been previously equilibrated with phosphate-buffered saline (pH 7.2). A linear gradient of NaCl (0—0.8 M) in phosphate-buffered saline (pH 7.2) was applied and 20 ml fractions were collected. The IL 2-containing fractions were pooled and polyethyleneglycol (MW 6000) added to a final concentration of 0.1% (w/v) to stabilize the IL 2.

### Chromatography on Procion®-Red Agarose (Fraction VI)

The pool of active fractions eluted from Blue Agarose was dialyzed against phosphate-buffered saline (pH 7.2) and loaded on a 10 ml Procion®-Red Agarose column (obtained from BRL), which has been previusly equilibrated with phosphate-buffered saline (pH 7.2). The column was then washed with phosphate-buffered saline (pH 7.2) and bound proteins were eluted by using a stepwise gradient of NaCl in phosphate-buffered saline (pH 7.2) with a starting salt concentration of 0.3 M NaCl and a final concentration of 1,0 M NaCl.

### High Performance Liquid Chromatography

A Micromeritics liquid chromatograph Model 700 B (Micromeritics, Norcross, Georgia) with a fixed wavelength detector and injector system was used. The chromatography was performed either in the presence or absence of sodium dodecyl sulfate/dithiothreitol. Under non-denaturing conditions, 1 ml of Blue Agarose-purified IL 2 was injected into a Micropak TSK 3000 SW column (from Varian, Sunnyvale, California) and eluted with 0.035 M sodium phosphate buffer (pH 6.8) at a flow rate of 0.8 ml/min. Fractions of 0.4 ml each were collected and 0.1% polyethyleneglycol (MW 6000) (w/v) was added to the fractions. The column was calibrated with bovine serum albumin (MW 68,000), chymotrypsinogen (MW 25,000), and ribonuclease A (MW 14,000), all obtained from Pharmacia. For analysis under denaturing conditions, 1 ml of Blue Agarose-purified IL 2 was treated with 1% sodium dodecyl sulfate and 10 mM dithiothreitol at 37°C for 1 hour and loaded onto the same column (previously equilibrated with 0.035 M sodium phosphate buffer (pH 6.8) containing 0.1% sodium dodecyl sulfate and 1 mM dithiothreitol). Marker proteins were pretreated in the same way and then used for column calibration.

### Isoelectrofocusing

Ten mls of the AcA Ultrogel® preparation of IL 2 were supplemented with 20% glycerol (v/v) and 2% Ampholine (v/v) (pH 3.5—10) (from LKB). A 5—60% glycerol density gradient, containing 0.1% polyethyleneglycol (MW 6000) (w/v) and 2% Ampholine (pH 3.5—10) ws layered into an isoelectrofocusing column (110 ml from LKB). The IL 2 sample was applied onto the isodense region of the gradient, followed by focusing for 24 hrs at 4°C using a constant power supply (Model 2103 from LKB). The terminal voltage was 2000 V and the terminal current 4 mA. Five ml fractions were collected and the pH determined in every fraction. All fractions were dialyzed against phosphate-buffered saline (pH 7.2) containing 0.1% polyethyleneglycol (MW 6000) (w/v) to remove the bulk of Ampholine and glycerol. The IL 2-containing fractions were pooled.

### Concanavalin A—Agarose Chromatography

One ml of Procion®-Red Agarose-purified IL 2 (500 U/ml) was loaded on a 2 ml concanavalin A—agarose column (from Pharmacia) equilibrated with 0.02 M sodium phosphate buffer (pH 7.2) containing 1 M NaCl and 1 mM each of $MgCl_2$, $MnCl_2$, and $CaCl_2$. The column was washed with the equilibration buffer and the proteins were eluted with the same buffer containing 0.1 M alpha-methyl-alpha-D-mannoside (obtained from Sigma).

### Wheat Germ Agglutinin Column Chromatography

The wheat germ agglutinin column (2 ml from Pharmacia) was equilibrated with phosphate-buffered saline (pH 7.2) and 1 ml of Procion®-Red Agarose-purified IL 2 was loaded onto the column. After washing with phospahte-buffered saline (pH 7.2) the column was eluted with phosphate-buffered saline (pH 7.2) containing 0.1 M N-acetyl-glucosamine (obtained from Sigma).

### Treatment with Neuraminidase

Blue Agarose-purified IL 2 (1000 U/ml) was adjusted to pH 5.0 with acetic acid and added to agarose-bound neuraminidase (from Clostridium perfringens, Type VI—A, supplied by Sigma). The mixture was incubated for 90 min at 37°C with gentle shaking. The neuraminidase-agarose was then removed by centrifugation and the pH of the IL 2-containing supernatant was adjusted to 7.8 with Tris base. A control sample was treated in the same way except that the neuraminidase-agarose was removed prior to the incubation.

### Protein Assay

The protein content of samples was measured using the Lowry technique (Lowry, O. H., N. J. Rosebrough, A. L. Farr and R. J. Randall, Protein Measurement with the Folin Phenol Reagent, J. Biol. Chem. 193:265 (1951)). For protein concentrations lower than 5 µg/ml, samples were subjected to sodium dodecyl sulfate-polyacrylamide gel electrophoresis; the protein bands were visualized by the silver staining technique (Merril, C. R., R. C. Switzer and M. L. Van Keuren, Trace Polypeptides in Cellular Extracts and Human Body Fluids Detected by Two-Dimensional Electrophoresis and a Highly Sensitive Silver Stain, Proc. Natl. Acad. Sci. U.S.A. 76:4335 (1979)); and the protein concentration estimated by comparison with known amounts of protein standards (soybean trypsin inhibitor and alpha-lactalbumin). Serial dilutions (200 ng to 2 ng) of these marker proteins were used.

## Sodium Dodecyl Sulfate-Polyacrylamide Gel Electrophoresis

The discontinuous Tris-glycine system of Laemmli (Laemmli, U.K., Cleavage of Structural Proteins During the Assembly of the Head of Bacteriophage Ty, Nature (Lond.) 227:680 (1970)) was used for 1.5-mm thick slab gels using a 5—20% gradient or a 15% of acrylamide. The samples were analyzed under both reduced (2% sodium dodecyl sulfate, 5% mercaptoethanol) and non-reduced (2% sodium dodecyl sulfate) conditions. After electrophoresis, gels were stained with Coomassie Brilliant Blue or by a silver nitrate method (Merril, C. R., R. C. Switzer and M. L. Van Keuren, Trace Polypeptides in Cellular Extracts and Human Body Fluids Detected by Two-Dimensional Electrophoresis and a Highly Sensitive Silver Stain, Proc. Natl. Acad. Sci. U.S.A. 76:4335 (1979)). Apparent molecular weights were determined using protein standards phosphorylase b (MW 94,000), bovine serum albumin (MW 68,000), ovalbumin (MW 43,000), carbonic anhydrase (MW 30,000), soybean trypsin inhibitor (MW 20,000) and alpha-lactalbumin (MW 14,500). After electrophoresis, the gels were sliced into 1-mm sections and proteins from each slice were eluted in 0.3 ml phosphate-buffered saline (pH 7.2). After 12—18 hours, the eluted materials were assayed for IL 2 activity.

## Assay for IL 2 Activity

For the IL 2 assay, 4000 murine IL 2-dependent cytotoxic T-cell line cells were grown in the presence of log 2 dilutions of putative IL 2-containing medium in 96-well microtiter plates (obtained from Costar, Cambridge, Massachusetts). The total volume in each well was 0.2 ml. Twenty-four hours later, 0.5 $\mu$Ci of 3H-thymidine (specific activity 20 Ci/mmole, supplied by New England Nuclear, Boston, Massachusetts) were added to each well. After 4 hours, the cells were harvested on glass fiber strips and 3H-thymidine incorporation measured in a liquid scintillation counter (from Packard, Downers Grove, Illinois). The IL 2 concentration in the experimental sample was then calculated by probit analysis (Gillis, S., and K. A. Smith, T-Cell Growth Factor: Parameters of Production and a Quantitative Microassay for Activity, J. Immunol. 120:2027 (1978)), using a standard containing 2 U/ml of IL 2. One unit/ml of IL 2 was defined as the quantity of IL 2 released in 48 hours culture medium conditioned by rat spleen cells (1 $\times$ 10$^6$/ml) stimulated by Con A (5 $\mu$g/ml) (Gillis, S., and K. A. Smith, T-Cell Growth Factor: Parameters of Production and a Quantitative Microassay for Activity, J. Immunol. 120:2027 (1978)).

High levels of IL 2 production were achieved by sequential stimulation of pooled peripheral blood lymphocytes from multiple donors. Peripheral blood lymphocytes were first stimulated by Sendai Virus for 12 hours. After a change of serum-free culture medium, these cells were restimulated by phytohemagglutinin for an additional 48 hours.

Sendai Virus or phytohemagglutinin alone stimulated the production of IL 2 at a 6—10 U/ml level, while sequential stimulation by Sendai Virus and phytohemagglutinin increased IL 2 production to 50—100 U/ml. An additional increase in IL 2 production, up to 200 U/ml, was achieved by costimulation with Daudi cells.

Lymphocyte-conditioned medium was precipitated with $(NH_4)_2SO_4$ at 80% saturation. This yielded an approximate 10-fold concentration of the proteins with high recovery of IL 2 activity.

See Table I.

Table I: <u>Purification of Human Interleukin 2</u>

| Fraction | | Total Protein (mg) | Total Activity (U) | Specific Activity (U/mg protein) | Purification (fold) | Yield (%) |
|---|---|---|---|---|---|---|
| I | Ly-CM[+] | 10,800 | 297,000 | 27 | 1 | 100 |
| II | $(NH_4)_2SO_4$-precipitate | 9,000 | 247,000 | 27 | | 84 |
| III | DEAE cellulose (DE 52) | 135 | 183,000 | 1,356 | 50 | 62 |
| IV | AcA 44 Ultrogel[R] | 40 | 145,000 | 3,625 | 135 | 49 |
| V | Blue Agarose | 0.96 | 87,680 | 91,333 | 3,382 | 30 |
| VI | Procion[R]-Red Agarose | 0.055[++] | 55,229 | 1,004,164 | 37,191 | 19 |

[+] The IL 2 activity in the Ly-CM (lymphocyte-conditioned medium) was 100 U/ml.

[++] Protein concentration was determined by densitometric comparison of the protein content in the samples with known amounts of protein standards as detailed herein.

Dialyzed $(NH_4)_2SO_4$ precipitate was placed on a diethylaminoethyl cellulose column (DE 52). IL 2 was eluted with a salt gradient from 0—0.3 M NaCl in 0.05 M Tris-HCl (pH 7.8) buffer. The IL 2 produced in the absence of Daudi cells eluted as a broad peak at low salt concentration (0—0.03 M NaCl), while the IL 2 produced by costimulation with Daudi cells eluted at salt concentrations between 0.03 and 0.08 M NaCl. See Figure 1. Under both conditions, the bulk of proteins were eluted at higher salt concentrations (0.01—0.3 M NaCl). This permitted a separation of IL 2 from the bulk of proteins in large scale batch preparations by using the 0.1 M NaCl wash of the diethylaminoethyl cellulose for elution of IL 2.

Anionic exchange chromatography on diethylaminoethyl cellulose achieved about 50-fold purification and approximately 75% of the loaded IL 2 activity may be recovered in this procedure. See Table I.

The material eluted from diethylaminoethyl cellulose may then be concentrated approximately 20-fold by dialysis against 50% polyethyleneglycol (MW 6000) (w/v) and loaded onto an AcA Ultrogel® column. The IL 2 produced in the absence of Daudi cells was eluted in fractions 42—52 as a single peak corresponding to a molecular weight of 26,000±4000 daltons. When IL 2 was produced by costimulation with Daudi cells, a major peak of activity was eluted on fractions 52—66, corresponding to a molecular weight of 13,000—18,000 daltons, while a minor peak of activity was found at 26,000 daltons. See Figure 2. The IL 2 activity-containing fractions were pooled.

This purification step achieved about a 2.7-fold increase in the specific activity and approximately 80% recovery of the loaded IL 2 activity. See Table I. This step also effectively removed proteins having molecular weights greater than 30,000 daltons. See Figure 5.

IL 2 bound strongly to Blue Agarose, while most proteins did not bind to the column, as shown in Figure 3. IL 2 was eluted from this column with 0.5—0.6 M NaCl and could be clearly separated from alpha-Interferon, which eluted at 0.05—0.4 M NaCl in phosphate buffer. See Figure 3. In this purification step the specific activity increased 25-fold, with 61% recovery of the loaded IL 2 activity. See Table I.

Procion®-Red Agarose has different binding properties from that of Blue Agarose (Thompson, S. T., and E. E. Stellwagen, Binding of Cibacron Blue F3GA to Proteins Containing the Dinucleotide Fold, Proc. Natl. Acad. Sci. U.S.A. 73:361 (1976); Watson, D. H., M. J. Harvey and P. D. Dean, The Selective Retardation of DADP⁺-Dependent Dehydrogenases by Immobilized Procion Red HE—3B, Biochem J. 173:591 (1978)). IL 2 was also bound strongly to this column and eluted as broad peak between 0.6—0.9 M NaCl in phosphate buffer with peak activity in the 0.7—0.8 M NaCl eluate. See Figure 4. This broad elution profile was suggestive of molecular heterogeneity of IL 2. A majority of the other proteins did not bind to Procion®-Red Agarose, and the rest eluted at low salt concentrations, as shown in Figure 4. The 0.7 M—0.8 M NaCl eluate pool was found, through silver staining of a 5—20% gradient gel (sodium dodecyl sulfate-polyacrylamide gel electrophoresis), to contain three molecular components with molecular weights of 14,500±2000, 16,000±1000 and 17,000±1000 daltons depending on the experimental condition used for the production of IL 2. See Figure 7.

The protein content of the preparation was measured by comparing the density of protein bands, visualized by silver staining, with serial dilutions of protein standards of known concentrations. Taking into account the limitation of this measurement, a specific activity of approximately $10^6 \pm 10\%$ U/mg protein and a final purification of 37,191-fold were calculated. The overall recovery of IL 2 was 19% after Procion®-Red Agarose chromatography (Fraction VI, Table I).

The strong binding of IL 2 to Procion®-Red Agarose also made this step very useful for concentrating IL 2 from diluted preparations using a two-step elution method with phosphate buffer containing 0.5 M and 1.0 NaCl. Under this condition, IL 2 activity was fully recovered in the 1.0 M NaCl eluate.

The IL 2 preparation from various steps of purification were subjected to sodium dodecyl sulfate-polyacrylamide gel electrophoresis analysis. Preparations obtained prior to the Blue Agarose chromatography (Fractions I—IV) were analyzed on a 5—20% gradient gel followed by Coomassie brilliant blue staining as shown in Figure 5. Preparations obtained after Blue Agarose chromatography and Procion®-Red Agarose chromatography were also analyzed on a 5—20% gradient gel followed by the highly sensitive silver staining method as shown in Figure 6.

When IL 2 was produced in the absence of Daudi cells and denatured, the Procion®-Red Agarose preparation showed only two active bands with MW of 16,000 and 17,000 daltons (Figure 6 (c)) under both reducing and non-reducing conditions. When Daudi cells were used as costimulator for IL 2 production, one active protein band with a MW of 14,500 daltons was observed (Figure 6(d)). If suboptimal concentrations of Daudi cells (less than $5 \times 10^5$/ml) were used, three protein bands with MW 14,500, 16,000 and 17,000 daltons were found.

To obtain a better resolution, the purified IL 2 was also analyzed on a 15% acrylamide gel. After staining, a molecular weight pattern similar to that obtained in the gradient gel was found. A parallel gel was sliced into 1-mm sections and proteins from each slice were eluted in phosphate-buffered saline (pH 7.2). IL 2 activity was found to be localized in slice numbers corresponding to molecular weights of 14,500, 16,000 and 17,000 daltons (Figure 7). Re-electrophoresis of the proteins present in all three slices with IL 2 activity showed one single band with molecular weight identical to that of the eluted band.

Blue Agarose-purified IL 2 , produced in the absence of Daudi cells, was incubated with 1% sodium dodecyl sulfate and 20 mM dithiothreitol at 37°C for 1 hour and applied to an high performance liquid chromatography gel filtration column. The column was eluted with buffer containing 0.1% sodium dodecyl sulfate and 1 mM dithiothreitol. As shown in Figure 8, sodium dodecyl sulfate denatured IL 2 was eluted in

10

the 17,000 daltons molecular weight region. Native IL 2, eluted with sodium dodecyl sulfate-free buffer, exhibited and apparent molecular weight of 26,000 daltons.

The IL 2 preparation obtained from the AcA 44 Ultrogel column was subjectd to isoelectrofocusing analysis using Ampholines with a broad pH range (pH 3.5—10). The IL 2 obtained without Daudi costimulation was focused with an approximate isoelectric point of 6.7. See Figure 9. The same pI was found if peripheral blood lymphocytes were stimulated in the presence of Daudi cells. The relatively broad focusing range observed, pH 6.5—7.5, was probably due to the reported molecular heterogeneity of IL 2 (Robb, R. J., and K. A. Smith, Heterogeneity of Human T-Cell Growth Factor(s) Due To Variable Glycosylation, Mol. Immunol. 18:1087 (1981)). The yield of IL 2 from the isoelectrofocusing column was approximately 30%. This method was, therefore, useful only for the biochemical characterization of IL 2, and not advantageous for preparative purification.

We were unable to detect any binding of IL 2 to either agarose-bound concanavalin A or to a wheat germ agglutinin column. Neuraminidase treatment of IL 2 did not affect its biological activity or its molecular weight pattern.

The mitogenicity on normal peripheral blood lymphocytes, the capacity to support the growth of murine and human cytotoxic T-cell line and the presence of other cytokines were studied in the purified IL 2. In order to test for the presence of phytohemagglutinin, the mitogenicity of the IL 2 preparations was studied on normal peripheral blood lymphocytes. A low level of mitogenic activity, about 5% of that present in lymphocyte-conditioned medium, was detected only in undiluted IL 2 preparations obtained from the diethylaminoethyl cellulose purification step. Blue Agarose- and Procion®-Red Agarose-purified IL 2 was completely free of mitogenic activity. IL 2 obtained from Blue Agarose and Procion®-Red Agarose chromatography reportedly very actively supports the long term growth of human and murine cytotoxic T-cell lines. Human cytotoxic T-cell line appear to require approximately 10 U/ml purified IL 2 for their optimum growth, while murine cytotoxic T-cell line are maximally stimulated at 2 U/ml. Procion®-Red Agarose preparations of IL 2 contained no detectable alpha-or gamma-Interferon, granulocyte-macrophage colony-stimulating activity, T-cell replacing factor, or thymocyte differentiating activity.

Lymphocyte-conditioned medium harvested after sequential stimulation of peripheral blood lymphocytes with Sendai Virus and phytohemagglutinin, and costimulation with Daudi cells, showed an IL 2 concentration of 100—200 U/ml. It was possible, therefore, to obtain a high IL 2 concentration in the absence of other potentially toxic costimulators such as PMA (Robb, R. J., A. Munck and K. A. Smith, T-Cell Growth Factor Receptors. Quantitation, Specificity, and Biological Relevance, J. Exp. Med. 154:1455 (1981); Mizel, S. G., and D. Mizel, Purification to Apparent Homogeneity of Murine Interleukin 1, J. Immunol. 126:834 (1981)), which could interfere with biological testing and clinical trials of purified IL 2.

The purification procedure disclosed introduces the chromatography on Blue Agarose and on Procion®-Red Agarose as two new purification steps for IL 2. Although it has been suggested that these dyes bind specifically to proteins containing the dinucleotide fold (Thompson, S. T., and E. E. Stellwagen, Binding of Cibacron Blue F3GA to Proteins Containing the Dinucleotide Fold, Proc. Natl. Acad. Sci. U.S.A. 73:361 (1976)), we have found no evidence for any effect of NAD+ or NADH on biological activity or biochemical behaviour of IL 2. The binding of IL 2 to these dyes may be due to the electrostatic or hydrophobic interactions. The use of these two steps permitted a 37,000-fold purification of IL 2 from medium conditioned in the presence of 0.25% bovine serum albumin, with a 19% overall recovery of IL 2 activity. All other purification methods, for both murine and human IL 2, have achieved neither a specific activity nor a yield comparable to those described here. Our purification procedure also avoided time consuming steps. This has made our procedure very useful for large scale purification of IL 2.

Lymphokines and other regulator molecules such as IL 1, (Mizel, S. G., and D. Mizel, Purification to Apparent Homogeneity of Murine Interleukin 1, J. Immunol. 126:834 (1981)), alpha- or gamma-Interferon, T-cell replacing factor, and CSF (Burgess, A. W., and D. Metcalf, The Nature and Action of Granulocyte Macrophage Colony Stimulating Factors, Blood 56:947 (1980)), have different capabilities of forming hydrophobic interactions. These properties were utilized to separate IL 2 from other lymphokines and factors which contaminate most partially purified IL 2 preparations. For example, alpha-Interferon co-purified with IL 2 during ion exchange chromatography and gel filtration steps, but was clearly separated from IL 2 by Blue Agarose chromatography. See Figure 3. After chromatography on Procion®-Red Agarose, the IL 2 preparation did not contained any detectable Interferon (alpha and gamma), granulocyte-macrophage colony-stimulating factor, T-cell replacing factor, or thymocyte differentiating activities. In addition, the Procion®-Red Agarose-purified IL 2 appeared to be free of any contaminating proteins (Figure 6).

Native IL 2 has been previously shown to exist in several molecular forms (Mier, J. W., and R. C. Gallo, Purification and Some Characteristics of Human T-Cell Growth Factor Phytohemagglutinin-Stimulated Lymphocyte Conditioned Media, Proc. Natl. Acad. Sci. U.S.A. 77:6134 (1980); Gillis, S., K. A. Smith and J. Watson, Biochemical Characterization of Lymphocyte Regulatory Molecules. II. Purification of a Class of Rat and Human Lymphokines, J. Immunol. 124:1954 (1980); Robb, R. J., and K. A. Smith, Heterogeneity of Human T-Cell Growth Factor(s) Due To Variable Glycosylation, Mol. Immunol. 18:1087 (1981)). Here we show that the methods used for IL 2 induction by peripheral blood lymphocytes can be responsible for the heterogeneity. Native IL 2 produced in the presence or absence of Daudi cells exhibited molecular weights of about 14,500 and 26,000 daltons, respectively (Figures 2 and 8). Both molecular forms could be obtained

11

by varying the concentration of costimulator cells. These results demonstrate that molecular weight differences reported by Mier *et al.,* (Mier, J. W., and R. C. Gallo, Purification and Some Characteristics of Human T-Cell Growth Factor From Phytohemagglutinin-Stimulated Lymphocyte Conditioned Media, Proc. Natl. Acad. Sci. U.S.A. 77:6134 (1980)) and Gillis *et al.,* (Gillis, S., K. A. Smith and J. Watson, Biochemical Characterization of Lymphocyte Regulatory Molecules. II. Purification of a Class of Rat and Human Lymphokines, J. Immunol. 124:1954 (1980)) were most likely due to different methods of IL 2 induction.

After denaturation by sodium dodecyl sulfate, the 26,000-dalton IL 2 exhibited a molecular weight of 16,000—18,000 daltons by high performance liquid chromatography gel filtration. Sodium dodecyl sulfate-polyacrylamide gel electrophoresis of this denatured form demonstrated the presence of two active bands with molecular weights of about 16,000 and 17,000 daltons, respectively. These results, together with those of Caplan *et al.,* (Caplan, B., C. Gibbs and V. Paetkau, Properties of Sodium Dodecyl Sulfate-Denatured Interleukin 2, J. Immunol. 126:1264 (1981)), indicate that, after sodium dodecyl sulfate denaturation, human and murine IL 2 exhibit similar molecular weights. The molecular weight of human IL 2 produced in the presence of Daudi cells was not affected by sodium dodecyl sulfate denaturation. Since native rat IL 2 has been reported to show a molecular weight of 15,000 daltons, (Gillis, S., K. A. Smith and J. Watson, Biochemical Characterization of Lymphocyte Regulatory Molecules. II. Purification of a Class of Rat and Human Lymphokines, J. Immunol. 124:1954 (1980); Smith, K. A., T-Cell Growth Factor, Immunological Rev. 51:337 (1981); Smith, K. A., P. E. Baker, S. Gillis and F. W. Ruscetti, Functional and Molecular Characteristic of T-Cell Growth Factor, Molecular Immunol. 17:579 (1980)), it appears that IL 2 activity is present in a polypeptide of about 14,500—17,000 daltons in all species studied. It is not known if these different molecular forms have different functions, such as preferential stimulation of T cell subsets. It is also not known whether the same T-cell will switch its IL 2 synthesis from 26,000 daltons to 14,500 daltons upon costimulation by Daudi cells, or whether different T-cell subsets are indeed responsible for the release of IL 2 in two molecular forms. Assuming two functional T-cell subsets, one subset would produce the 26,000 MW IL 2 in response to phytohemagglutinin alone, while the second subset would require phytohemagglutinin as primary stimulus and costimulation by Daudi cells as a second signal for the production of 14,500 MW IL 2 . Daudi cells express HLA—DR antigens and Fc receptors. Both of these surface molecules have been implicated in the augmentation of the IL 2 response (Palacios, R. and G. Moller, HLA—DR Antigens Sender Reoting T-Cells Sensitive to Interleukin-2 and Induce Production of the Growth Factor in the Autologous Mixed Lymphocyte Reaction, Cellular Immunol. 63:143 (1981); Shimizu, S., R. T. Smith, M. A. Norcross and V. C. Mario, Mechanismus Controlling TCGF Production by Cloned Sublines of EL—4 azg$^r$ in Response to Stimulation by Anti Thy—1 Antibody, J. Immunol. 128:296 (1982)). The effect of Daudi cells on the IL 2 response, however, does appear to be more complicated than previously suggested (Palacios, R., and G. Moller, HLA—DR Antigens Render Resting T-Cells Sensitive to Interleukin-2 and Induce Production of the Growth Factor in the Autologous Mixed Lymphocyte Reaction, Cellular Immunol. 63:143 (1981); Shimizu, S., R. T. Smith, M. A. Norcross and V. C. Mario, Mechanisms Controlling TCGF Production by Cloned Sublines of EL—4 azg$^r$ in Response to Stimulation by Anti Thy—1 Antibody, J. Immunol. 128:296 (1982)), in view of (a) the shift in molecular weight of IL 2 from 26,000 to 14,500 daltons induced by Daudi cells; (b) the augmentation if IL 1-independent IL 2 production as seen in the human lymphoblastic cell line Jurkat (Venuta, S., R. Mertelsmann, K. Welte, S. P. Feldman, C. Y. Wang and M. A. S. Moore, Production and Regulation of Interleukin-2 in Human Lymphoblastic Leukemias Studied with T-Cell Monoclonal Antibodies (submitted)); and (c) the superinduction of IL 2 in human lymphoblastic leukemic cells by co-stimulation with Daudi cells (Venuta, S., R. Mertelsmann, K. Welte, S. P. Feldman, C. Y. Wang and M. A. S. Moore, Production and Regulation of Interleukin-2 in Human Lymphoblastic Leukemias Studied with T-Cell Monoclonal Antibodies (submitted)). This observed molecular and possibly functional heterogeneity of a growth stimulator are not unique for IL 2 since another group of cytokines, the granulocyte-macrophage colony-stimulating factors, have been shown to exhibit different molecular characteristics depending on the producer cell type, which characteristics were also associated with preferential effects on subsets of the granulocyte-macrophage cell lineage (Burgess, A. W., and D. Metcalf, The Nature and Action of Granulocyte Macrophage Colony Stimulating Factors, Blood 56:947 (1980)). The possibility that different T-cell subsets are responsible for the production of the two IL 2 forms is supported by recent reports on the molecular weights of IL 2 produced by human leukemic cells. We have previously shown that non-T non-B lymphoblastic leukemias produce the 26,000 MW form of IL 2 after phytohemagglutinin stimulation (Venuta, S., R. Mertelsmann, K. Welte, S. P. Feldman, C. Y. Wang and M. A. S. Moore, Production and Regulation of Interleukin-2 in Human Lymphoblastic Leukemias Studied with T-Cell Monoclonal Antibodies (submitted)), while Friedman *et al.* (Friedman, S. M., G. Thompson, J. P. Halper and D. M. Knowles, OT-CLL: A Human T-Cell Chronic Lymphocytic Leukemia that Produces IL 2 in High Titer, J. Immunol. 128:935 (1982)) recently reported that cells obtained from a patient with T-cell chronic lymphocytic leukemia, stimulated under identical conditions, released the 14,500 MW form of IL 2.

The reason for the molecular heterogeneity of the sodium dodecyl sulfate-denatured and native IL 2 remains to be explored. A variable degree of glycosylation may provide an explanation of this phenomenon (Robb, R. J., and K. A. Smith, Heterogeneity of Human T-Cell Growth Factor(s) Due To Variable Glycosylation, Mol. Immunol. 18:1087 (1981); Clark-Lewis, I., and J. W. Schrader, Biochemical Characterization of Regulatory Factors Derived from T-Cell Hybridomas and Spleen Cells. II. Evidence For

# 0 092 163

Glycosylation of T-Cell Growth Factor, T-Cell Replacing Factor, and Granulocyte-Macrophage Colony-Stimulating Factor, J. Immunol. 128:180 (1982)). Robb *et al.* (Robb, R. J., and K. A. Smith, Heterogeneity of Human T-Cell Growth Factor(s) Due To Variable Glycosylation, Mol. Immunol. 18:1087 (1981)) have shown that neuraminidase, glycosidases and inhibitors of glycosylation can reduce the heterogeneity of IL 2 produced by tonsil lymphocytes. However, we were not able to affect the molecular weight of IL 2 by neuraminidase treatment, nor were we able to detect any binding of IL 2 to immobilized lectins (concanavalin A and wheat germ agglutinin). The lack of binding of IL 2 to lectins has been previously reported by Mier *et al.* (Mier, J. W., and R. C. Gallo, Purification and Some Characteristics of Human T-Cell Growth Factor From Phytohemagglutinin-Stimulated Lymphocyte Conditioned Media, Proc. Natl. Acad. Sci. U.S.A. 77:6134 (1980)). The differences between these results are probably due to the different methods of induction and purification of IL 2, which appear to affect the biochemical characteristics of IL 2.

The purification steps described herein produced IL 2 with a specific activity of about $10^6$ U/mg protein. Since the lowest molecular weight of an active IL 2 polypeptide was 14,500 daltons, it could be calculated that 1 U/ml of IL 2 was equivalent to a molar concentration of $7 \times 10^{-11}$ M. An IL 2 concentrations of $1.4 \times 10^{-11}$ M or approximately $4 \times 10^5$ molecules/cell was required for half maximum stimulation of murine cytotoxic lymphocytes. Similar values have been reported by Mizel for IL 1 (Mizel, S. G., and D. Mizel, Purification to Apparent Homogeneity of Murine Interleukin 1, J. Immunol. 126:834 (1981)).

The highly purified IL 2 may be used to investigate the biological effects of IL 2, both in vitro and in vivo. In preliminary experiments, we have been able to partially restore, in vitro, the response of T cells of a patient with Nezeloff's syndrome in both the allogeneic mixed lymphocyte reaction (MLR) and in cell-mediated lympholysis (CML) by addition of human Procion®-Red Agarose-purified IL 2 (Fraction VI). These studies should contribute to the understanding of normal human lymphocyte function, immunodeficiency syndromes, and the pathophysiology of human lymphoblastic leukemias (Gillis, S., R. Mertelsmann, B. Clarkson and M. A. S. Moore, Correlation of Elevated Terminal Transferase Activity (TdT) with T-Cell Growth Factor (TCGF) in Human Leukemia Cells, AACR, Abstract No. 955, p. 238 (1980); Venuta, S., R. Mertelsmann, K. Welte, S. P. Feldman, C. Y. Wang and M. A. S. Moore, Production and Regulation of Interleukin-2 in Human Lymphoblastic Leukemias Studied with T-Cell Monoclonal Antibodies (submitted)).

IL 2 is useful in a variety of in vitro systems, including the continuous growth of antigen specific cytotoxic T-cells and the establishment of IL 2 dependent long-term cell line from patients with certain t-cell neoplasias. It is also feasible to grow autologous tumor-specific human T-cells in large quantities in vitro and then transfuse these cells in vivo with a therapeutic result. Further uses to be considered are the establishment of long-term cultures of normal T-cells for drug testing and carcinogenicity tests.

Animal models that have been used to explore therapeutic uses of crude conditioned media containing IL 2 are the nude mouse (T-cell immunodeficiency) and the mouse immunosuppressed by cytoxan. Preliminary results suggest that the immune response can be normalized by these media (Lipsick et al., Proc. Natl. Acad. Sci. 78:2398 (1981); Merluzzi et al., Cancer Res. 41:850 (1981)). Preliminary studies using our own material suggest that the active ingredient in fact is IL 2.

Further studies in both mice (Miller et al., Eur. J. Immunol. 11:751 (1981)) and men (Gillis *et al.*, J. Clin. Invest. 67:937 (1981)) have shown that aging and its associated immune defects might be related to a defect in IL 2 production.

More recently, Lopez-Botet *et al.*, J. Immunol. 128:679 (1982), evaluated patients with severe combined immunodeficiency, Wiskott-Aldrich Syndrome, immunodeficiencies with hyper IgM, x-linked agammaglobulinemia, and common variable immunodeficiency for proliferative response to phytohemagglutinin with and without addition of mitogen-free supernatants containing IL 2. The hyporesponse to phytohemagglutinin in this group of patients was associated with a defect in the production of IL 2.

We have found that the defect of the proliferative response of T-cells in patients with Nezeloff Syndrome, in immunodeficiency associated with (a) Kaposi's Syndrome in homosexuals, (b) Hodgkin's disease, and (c) chemotherapy can almost be completely normalized in vitro by additions of homogeneous IL 2.

Based on these encouraging results we have submitted a phase I trial of IL 2 in patients with congenital and acquired immunodeficiency syndrome. So far no untoward reactions have been seen in one patient with Nezeloff's Syndrome (maximum dose level reached 100 U/day s.c.) and one normal volunteer (one of us, 1000 U/day s.c.).

We anticipate extensive clinical trials and therapeutic potential for patients with congenital immunodeficiency syndromes, Hodgkin's disease (T-cell defect frequent), Kaposi's Syndrome, immunosuppression associated with cancer, and immunosuppression associated with chemotherapy and/or radiation therapy; and for aged individuals as well as patients with other immunodeficient states.

We believe that IL 2 alone or in combination with other lymphokines might become the treatment of choice for several of the above listed syndromes, in a similar fashion as parenteral insulin has become the specific therapy for diabetes mellitus.

Further use is anticipated in IL 2-dependent neoplasia such a ALL (hypothetical) and neoplasias of mature T-cells (proven). Modification of the IL 2 molecule either chemically or through addition of cytotoxic substances to the IL 2 molecule may allow selective elimination of IL 2-dependent neoplastic cells through blockage of receptor sites or cell type specific delivery of toxic substances to IL 2 responder cells. This approach might also be helpful in selectively eliminating IL 2 responder cells in cases with over-reactive immunity, such as in autoimmune disorders.

## Claims

1. A process for purifying human Interleukin 2 (IL 2), a proteinaceous physiologically active substance, comprising the steps:

(a) stimulating IL 2 production in a lymphocyte-conditioned medium;

(b) concentrating the proteinaceous components of said step (a) medium by precipitation of same;

(c) separating salts from said concentrated proteinaceous components via dialysis;

(d) separating some non-specific proteinaceous material from the concentrated proteinaceous components of step (c) via anion exchange;

(e) effecting molecular weight separation of the remaining concentrated proteinaceous components by gel filtration into (1) IL 2-containing molecular weight proteinaceous material and (2) non-specific molecular weight proteinanceous material; and

(f) separating the step (e) (1) material into IL 2-containing proteinaceous material and non-specific proteinaceous material of about the same molecular weight via separation comprising a plurality of hydrophobic, chromatographic separations characterized by that at least one of said hydrophobic separations of step f) comprising chromatography of IL 2-containing proteinaceous material on Blue Agarose and/or wherein at least one of said hydrophobic chromatographic separations comprises chromatography of IL 2-containing proteinaceous material on Procion®-Red Agarose.

2. A process according to claim 1 characterized by, that the lymphocytes are stimulated by irradiated DAUDI-cells.

## Patentansprüche

1. Verfahren zur Reinigung von humanem Interleukin 2 (II 2), einer eiweißartigen, physiologisch aktiven Substanz, wobei das Verfahren folgende Schritte umfaßt:

a) Stimulierung der II 2 Produktion in einem Medium, das für Lymphozyten konditioniert ist;

b) Anreicherung der eiweißhaltigen Komponenten des genannten Mediums des Schrittes (a) durch Präzipitation;

c) Abtrennen von Salzen von den genannten konzentrierten eiweißartigen Komponenten durch Dialyse;

d) Abtrennen eines Teils des nicht-spezifischen eiweißartigen Materials von den konzentrierten, eiweißartigen Komponenten des Schrittes (c) durch Anionen-Austausch;

e) Durchführen einer Molekulargewichts-Auftrennung in (1) II 2 enthaltendes, im Molekulargewicht entsprechendes, einweißartiges Material und (2) vom Molekulargewicht her nicht spezifisches, eiweißartiges Material; und

f) Auftrennung des Materials (1) aus Schritt (e) in II 2 enthaltendes eiweißartiges Material und nicht spezifisches eiweißartiges Material von etwa dem gleichen Molekulargewicht durch Separation, die eine Vielzahl von hydrophobischen, chromatographischen Trennschritten umfaßt, dadurch gekennzeichnet, daß wenigstens eine der hydrophobischen Trennungen gemäß Schritt (f) eine Chromatographie von II 2 enthalten en Material auf Blauer Agarose ("Blue Agarose") umfaßt, und/oder worin wenigstens einer der hydrophobischen chromatographischen Trennschritte die Chromatographie von II 2-enthaltenen eiweißartigen Material auf Procion® Roter Agarose ("Red Agarose") umfaßt.

2. Verfahren nach Anspruch 1, dadurch gekennzeichnet, daß die Lymphozyten durch bestrahlte DAUDI-Zellen stimuliert werden.

## Revendications

1. Procédé pour la purification d'Interleukine humaine 2 (IL 2), qui est une substance protéinique physiologiquement active, comprenant les opérations de

a) stimulation de la production d'IL 2 dans un milieu conditionné par lymphocytes;

b) concentration des composants protéiniques dudit milieu de l'opération (a), par précipitation de ces composants;

c) séparation des sels desdits composants protéiniques concentrés, par dialyse;

d) séparation des matières protéiniques non spécifiques des composants protéiniques concentrés de l'opération (c), par échange d'anions;

e) exécution d'une séparation basée sur la masse moléculaire des composants protéiniques concentrés restants, par filtration de gel, en (1) matière protéinique de masse moléculaire contenant IL 2 et (2) matière protéiniques de masse moléculaire non spécifique; et

f) séparation de la matière (1) de l'opération (e) en matière protéinique contenant IL 2 et matière protéinique non spécifique sensiblement de même masse moléculaire, par séparation comprenant une pluralité de séparations chromatographiques hydrophobes,

caractérisé en ce qu'au moins une des dites séparations hydrophobes de l'opération (f) comprend une

chromatographie de la matière protéinique contenant IL 2 sur Agarose Bleu, et/ou en ce qu'au moins une des dites séparations chromatographiques hydrophobes comprend une chromatographie de la matière protéinique contenant IL 2 sur Procion®-Agarose Rouge.

2. Procédé suivant la revendication 1, caractérisé en ce que les lymphocites sont stimulés par des cellules DAUDI irradiées.

F I G. I

FIG.2

F I G . 3

FIG.4

FIG.5

FIG.6

FIG.7

FIG. 8

IEF PBL

FIG. 9

0 092 163